Europäisches Patentamt

(19) European Patent Office

Office européen des brevets

(11) **EP 0 683 152 B1**

(12) **EUROPEAN PATENT SPECIFICATION**

(45) Date of publication and mention
of the grant of the patent:
**06.08.1997 Bulletin 1997/32**

(51) Int. Cl.$^6$: **C07C 53/08**, C07C 31/26,
C07C 59/08, B01J 39/04

(21) Application number: **95111207.7**

(22) Date of filing: **14.11.1991**

(54) **Method of separating and purifying mannitol**

Verfahren zur Abtrennung und Reinigung von Mannitol

Procédé pour séparer et purifier le mannitol

(84) Designated Contracting States:
**CH ES FR GB IT LI NL**

(30) Priority: **15.11.1990 JP 307147/90**

(43) Date of publication of application:
**22.11.1995 Bulletin 1995/47**

(62) Document number(s) of the earlier application(s) in
accordance with Art. 76 EPC:
**91119453.8 / 0 486 024**

(73) Proprietors:
• **SUMITOMO HEAVY INDUSTRIES, LTD
Tokyo 100 (JP)**
• **KATOH KAGAKU KABUSHIKIGAISHA
Chita-gun, Aichi-ken (JP)**

(72) Inventors:
• **Itoh, Yoshikuni
Chita-gun, Aichi-ken (JP)**
• **Tanaka, Akira
Hiratsuka-shi, Kanagawa-ken (JP)**
• **Araya, Hiroshi
Kamakura-shi, Kanagawa-ken (JP)**

(74) Representative: **Fiener, Josef
Patentanwälte
Kahler, Käck, Fiener et col.,
P.O. Box 12 49
87712 Mindelheim (DE)**

(56) References cited:
**CH-A- 388 282**

• **ASSOCIATION OF OFICIAL ANALYTICAL
CHEMISTS JOURNAL, vol. 67, no. 4, July 1984 -
August 1984 pages 710-714, MCFEETERS ET
AL. 'Liquid chromatographic analysis of sugars,
acids, and ethanol in lactic acid vegetable
fermentations'**
• **BULLETIN DE LA SOCIETE CHIMIQUE DE
FRANCE, 1963 PARIS, FR, pages 350-355, A.J.
COURTOISIER ET AL. 'Etude de la séparation
par chromatographie d'échange d'ions et du
dosage des acides lactique, malique et tartrique.
'**

**Description**

BACKGROUND OF THE INVENTION

The present invention relates to a method of separating and collecting mannitol, acetic acid and lactic acid individually from a mixture of these ingredients. Still more particularly, the present invention relates to a method of separating acetic acid, lactic acid and mannitol, in which acetic acid and lactic acid obtained as by-products in the mannitol fermentation can be collected individually at the same time.

Being in the form of a white crystal or powder, mannitol has a degree of sweetness 30-40% that of sucrose, cooling sweetness, and very low hygroscopicity. Mannitol is generally used as a diluent or filler of pharmaceuticals and preparations. For example, mannitol is used for intermediates of hypotensive drugs and in producing electrolytic condensers, as well as used for surface tack eliminators for chewing gums and candies, being an industrially useful substance.

Examples of methods of producing mannitol generally include a method of collecting mannitol from natural substances e.g., mannan, an ingredient of seaweeds, by extracting it with hot ethanol [as described in ENCYCLOPAEDIA CHIMICA, Vol.8, pp.924-925(1968)]; and an industrial method of reducing fructose or sucrose catalytically [as described in Shokuhin Tenkabutu Binran, p.387 (1968)]. A variety of methods of producing mannitol, such as a chemical synthesis, a fermentation method and an enzyme method are described in Japanese Laid Open Patent Application (Kokai) No. 239995/87.

Recently, mannitol has been produced by reducing sucrose under high pressure, separating sorbitol prepared in this process as a by-product using an ion exchange resin, and crystallizing the obtained material. However, this method has a drawback in which a unit cost is relatively high.

A microorganism which produces mannitol is known previously. For example, a method of preparing mannitol from a fermented medium in which a microorganism was cultured is described in ENCYCLOPAEDIA CHIMICA, Vol.8, pp.924-925 (1968). This method is reported by W. H. Peterson in 1920. In the fermentation method, fructose is reduced by the reductive pyridine nucleotide, which derived from the hexose monophosphate shunt by $\alpha$-hetero lactic acid bacteria, to form mannitol. In general, it is thought that the reaction is proceeded according to the following process.

2 fructose + 1 glucose or fructose $\rightarrow$ 2 mannitol + lactic acid + acetic acid + carbon dioxide + water.

Today microorganisms producing mannitol are known to be lactic acid bacteria, yeasts and fungi. Examples of lactic acid bacteria include Lactobacillus brevis, Leuconostoc mesenteroides, Lactobacillus fermentum, Leuconostoc dextrancam and the like. Examples of yeasts include Saccaromyces sake, genus Torulopsis, Cryptococcus neoformans, Candida lipolitica and the like. Examples of fungi include Ascomycetes such as genus Aspergillus and genus Penicillium, and the like.

No economical method has been available for separating individually mannitol, lactic acid and acetic acid useful in the industry from the fermented medium containing mannitol, lactic acid and acetic acid, which are produced at the same time during fermentation by the above mentioned lactic acid bacteria.

Part of mannitol can be collected from the medium fermented by these lactic acid bacteria, which contain mannitol, lactic acid and acetic acid, using the conventional methods, though it was difficult to separate individually mannitol, lactic acid and acetic acid in the residual medium by crystallization.

Mannitol can be also collected by adsorbing lactic acid and acetic acid, organic acids, to a strongly basic anion exchange resin of carbonate type (e.g., Amberlite™ IRA-400).

However, no methods have been available for separating lactic acid and acetic acid into each ingredient and collecting individually mannitol, lactic acid and acetic acid finally, even though mannitol, a sugar, could be separated from lactic acid and acetic acid, organic acids. Since organic acids such as lactic acid and acetic acid have not been separated previously, it was generally thought that organic acids could not be separated individually except that an organic acid had extremely different properties from other organic acids.

Thus it has been desired that a method should be available, by which lactic acid and acetic acid produced as by-products in the cultured medium obtained by culturing mannitol-producing bacteria are collected effectively.

SUMMARY OF THE INVENTION

The present invention is accomplished to overcome the above mentioned problems. The present inventors investigated intensively to find an industrial method of separating mannitol, lactic acid and acetic acid from the fermented medium containing these compounds obtained by the fermentation using a lactic acid bacterium.

Thus the present invention provides a method of preparing and collecting each ingredient from a mixture of mannitol, acetic acid and lactic acid by chromatography using a strongly acidic cation exchange resin.

In the method of separating and collecting mannitol from the fermented medium by the mannitol fermentation, not only mannitol but also pure lactic acid and acetic acid can be collected individually at the same time by using a strongly acidic cation exchange resin. As a result, the production cost of mannitol to be produced can be reduced, and the by-products can be also utilized effectively.

DETAILED DESCRIPTION OF THE INVENTION

The embodiment of the present invention is a method comprising: obtaining first an acetic acid fraction by eluting acetic acid from a mixture of mannitol, acetic acid and lactic acid by chromatography using a strongly acidic cation exchange resin; obtaining a mixed fraction of lactic acid and mannitol by eluting lactic acid and mannitol; obtaining mannitol as crystal by condensing the mixed fraction of lactic acid and mannitol to crystallize mannitol; and obtaining lactic acid as calcium lactate by adding calcium hydroxide to the residual solution in which the mannitol crystal was removed.

The mixture of mannitol, acetic acid and lactic acid can be obtained by culturing a lactic acid bacterium in a medium containing fructose or a mixture of fructose and glucose as a raw sugar material.

The present invention is illustrated further in detail in the following.

The present inventors removed a crystal of mannitol which was part of mannitol and obtained a filtrate; then separated and collected each ingredient from a solution containing mannitol, acetic acid and lactic acid by chromatography using a strongly acidic cation exchange resin to find that acetic acid is first eluted, and then mannitol and lactic acid almost at the same time; then accomplished the method of separating and collecting mannitol, acetic acid and lactic acid individually by separating the fractions of mannitol and lactic acid by crystallization.

Separation by chromatography using ion exchange resins is a method of separating two or more ingredients in a raw material on the basis of the difference of adsorption capacities between the ion exchange resins and the ingredients, being known to be of the fixed bed type and the moving bed type.

Examples of strongly acidic cation exchange resins include a resin such as LES™-200 (Duolite), which is a copolymer of styrene divinylbenzene, introduced by sulfonic acid groups, and the like.

The strongly acidic cation exchange resin according to the present invention has generally exchanging groups of sodium type because sodium hydroxide (NaOH) is used to adjust the pH in the mannitol fermentation processes before the fractionation by chromatography. Also, can be used the strongly acidic cation exchange resin having exchanging groups of calcium type. Lactic acid tends to be eluted more lately in the separation by the calcium type than in that by the sodium type, but elution of the other ingredients is not affected by both types.

The present invention relates to a method comprising: obtaining first an acetic acid fraction by eluting acetic acid from a mixture of mannitol, acetic acid and lactic acid by chromatography using a strongly acidic cation exchange resin; obtaining a mixed fraction of lactic acid and mannitol by eluting lactic acid and mannitol; obtaining mannitol by condensing the mixed fraction of lactic acid and mannitol to crystallize mannitol; and obtaining lactic acid as calcium lactate by adding calcium hydroxide to the residual solution in which the mannitol crystal was removed.

The present invention also relates to a method comprising: condensing a mixture of mannitol, acetic acid and lactic acid which contains a mannitol richly in order to crystallize part of mannitol to obtain a condensed solution; subjecting the condensed solution to filtration to remove a crystal of mannitol which is part of a mannitol and to obtain a filtrate; obtaining first an acetic acid fraction by eluting acetic acid from the filtrate containing mannitol, acetic acid and lactic acid by chromatography using a strongly acidic cation exchange resin; obtaining a mixed fraction of lactic acid and mannitol by eluting lactic acid and mannitol; obtaining mannitol as a crystal by condensing the mixed fraction of lactic acid and mannitol to crystallize mannitol; and obtaining lactic acid as calcium lactate by adding calcium hydroxide to the residual solution in which the mannitol crystal was removed.

The above mentioned mixture of mannitol, acetic acid and lactic acid is generally obtained in the cultured medium by culturing a microorganism having a capacity of producing lactic acid in a medium containing fructose or a mixture of fructose and glucose as a raw sugar material. Examples of such a microorganism include Lactobacillus brevis (ATCC 8287) , Leuconostoc mesenteroides (ATCC 9135) and Lactobacillus sp. B001 (deposited in Fermentation Research Institute Agency of Industrial Science and Technology, FERM BP-3158) which are available.

A strongly acidic cation exchange resin is preferably of a sodium type or of a calcium type. In a method of separating and purifying mannitol according to the present invention, a mixture of mannitol, acetic acid and lactic acid obtained from a process of mannitol fermentation is used as an original solution of filtrate in which part of the mannitol as a crystal was fractionated and removed by preliminary condensation and crystallization.

When the original solution is applied to a column packed with a strongly acidic cation exchange resin and eluted by water as an eluent, salts having low molecular weights are first eluted on the basis of the differences of adsorption capacities between the strongly acidic cation exchange resin and the salts. Acetic acid is then eluted (the acetic acid fraction), and a mixture of mannitol and lactic acid is eluted finally (the mannitol and lactic acid fraction). Mannitol is collected as a crystal by condensation and crystallization of the mannitol and lactic acid fraction, and then lactic acid is collected as calcium lactate by adding calcium hydroxide to the filtrate.

Example 1

A cultured medium containing mannitol, acetic acid and lactic acid, which was obtained by culturing <u>Lactobacillus sp.</u> B001 (FERM BP-3158), was filtered, and then the resulting filtrate was concentrated to crystallize mannitol. One hundred grams of mannitol, 100 g of lactic acid and 70 g of acetic acid were contained in 500 ml of the supernatant in which a crystal of mannitol was removed by filtration.

Five hundred milliliters of the mixture were applied to a column (10 cm diameter × 1 m length) packed by a sodium type strongly acidic cation exchange resin LES™-200 (a degree of crosslinking of 8 %), and subjected to elution with water.

A eluent which was eluted 40 to 51 minutes after initiation of the elution at a flow rate of 78 ml/min was collected as an acetic acid fraction. A eluent which was eluted 60 to 76 minutes after initiation of the elution was also collected as a mannitol-lactic acid fraction. A eluent which was eluted 51 to 60 minutes after initiation of the elution was returned to the original solution because acetic acid and lactic acid, organic acids, were mixed in the eluent. Table 1 shows the results obtained from analysis of these fractions by high performance liquid chromatography.

Table 1

| Yield (%) in Example 1 | | | |
|---|---|---|---|
| fraction | acetic acid | mannitol | lactic acid |
| acetic acid | 89 | 4 | 0 |
| mannitol-lactic acid | 0 | 88 | 89 |

In the acetic acid fraction, 89% of acetic acid was collected relative to the applied amount, with 4% of mannitol as impurity and without lactic acid. On the other hand, in the mannitol-lactic acid fraction 88% of mannitol and 89% of lactic acid were collected without acetic acid.

With respect to separation of mannitol and lactic acid, the mannitol-lactic acid fraction was condensed to crystallize mannitol, and filtered to collect a mannitol crystal. Calcium hydroxide was then added to the filtrate to precipitate lactic acid as calcium lactate, and the calcium lactate was collected by condensing the filtrate.

Example 2

The same original solution as in Example 1 was applied to a column packed by a sodium type strongly acidic cation exchange resin LES™-200, and subjected to elution with water.

A eluent which was eluted 40 to 51 minutes after initiation of the elution at a flow rate of 78 ml/min was collected as an acetic acid fraction. A eluent which was eluted 60 to 86 minutes after initiation of the elution was also collected as a mannitol-lactic acid fraction.

Table 1 shows the results obtained from the analysis by the same method as in Example 1.

Table 2

| Yield (%) in Example 2 | | | |
|---|---|---|---|
| fraction | acetic acid | mannitol | lactic acid |
| acetic acid | 89 | 4 | 0 |
| mannitol-lactic acid | 0 | 88 | 98 |

**Claims**

1. A method of separating acetic acid, lactic acid and mannitol individually comprising:

obtaining first an acetic acid fraction by eluting acetic acid from a mixture of mannitol, acetic acid and lactic acid by chromatography using a strongly acidic cation exchange resin;
obtaining a mixed fraction of lactic acid and mannitol by eluting lactic acid and mannitol;

obtaining mannitol as a crystal by condensing the mixed fraction of lactic acid and mannitol to crystallize mannitol; and
obtaining lactic acid as calcium lactate by adding calcium hydroxide to the residual solution in which the mannitol crystal was removed.

2. A method of separating acetic acid, lactic acid and mannitol individually comprising:

condensing a mixture of mannitol, acetic acid and lactic acid in order to crystallize part of mannitol to obtain a concentrated solution;
subjecting the condensed solution to filtration to remove a crystal of mannitol which is part of a mannitol ingredient and to obtain a filtrate;
obtaining first an acetic acid fraction by eluting acetic acid from the obtained filtrate containing mannitol, acetic acid and lactic acid by chromatography using a strongly acidic cation exchange resin;
obtaining a mixed fraction of lactic acid and mannitol by eluting lactic acid and mannitol;
obtaining mannitol as a crystal by condensing the mixed fraction of lactic acid and mannitol to crystallize mannitol; and
obtaining lactic acid as calcium lactate by adding calcium hydroxide to the residual solution in which the mannitol crystal was removed.

3. A method of separating acetic acid, lactic acid and mannitol of Claim 1 or 2 individually, wherein the strongly acidic cation exchange resin is of a sodium type.

4. A method of separating acetic acid, lactic acid and mannitol of Claim 1 or 2 individually, wherein the strongly acidic cation exchange resin is of a calcium type.

**Patentansprüche**

1. Verfahren zur individuellen Trennung von Essigsäure, Milchsäure und Mannitol, umfassend:

zuerst Erhalten einer Essigsäurefraktion durch Eluieren von Essigsäure von einem Gemisch von Mannitol, Essigsäure und Milchsäure durch Chromatographie unter Verwendung eines stark sauren Kationenaustauscherharzes;
Erhalten einer gemischten Fraktion von Milchsäure und Mannitol durch Eluieren von Milchsäure und Mannitol;
Erhalten von Mannitol als Kristall durch Kondensieren der gemischten Fraktion von Milchsäure und Mannitol, um Mannitol zu kristallisieren; und
Erhalten von Milchsäure als Calciumlactat durch Zugabe von Calciumhydroxid zu der zurückbleibenden Lösung, von der das Mannitolkristall entfernt wurde.

2. Verfahren zur individuellen Trennung von Essigsäure, Milchsäure und Mannitol, umfassend:

Kondensieren eines Gemischs von Mannitol, Essigsäure und Milchsäure, um einen Teil des Mannitols zu kristallisieren, um eine konzentrierte Lösung zu erhalten;
Unterziehen der kondensierten Lösung einer Filtration, um einen Mannitolkristall zu entfernen, der Teil eines Mannitolbestandteils ist, und um ein Filtrat zu erhalten;
Erhalten von zuerst einer Essigsäurefraktion durch Eluieren von Essigsäure vom erhaltenen Filtrat, das Mannitol, Essigsäure und Milchsäure enthält, durch Chromatographie unter Verwendung eines stark sauren Kationenaustauscherharzes;
Erhalten einer gemischten Fraktion von Milchsäure und Mannitol durch Eluieren von Milchsäure und Mannitol;
Erhalten von Mannitol als Kristall durch Kondensieren der gemischten Fraktion von Milchsäure und Mannitol, um Mannitol zu kristallisieren; und
Erhalten von Milchsäure als Calciumlactat durch Zugabe von Calciumhydroxid zu der zurückbleibenden Lösung, von der der Mannitolkristall entfernt wurde.

3. Verfahren zur individuellen Trennung von Essigsäure, Milchsäure und Mannitol nach Anspruch 1 oder 2, wobei das stark saure Kationenaustauscherharz vom Natrium-Typ ist.

4. Verfahren zur individuellen Trennung von Essigsäure, Milchsäure und Mannitol nach Anspruch 1 oder 2, wobei das stark saure Kationenaustauscherharz vom Calcium-Typ ist.

**Revendications**

1. Procédé de séparation un par un d'acide acétique, d'acide lactique et de mannitol comprenant :

   l'obtention dans un premier temps d'une fraction d'acide acétique par élution de l'acide acétique à partir d'un mélange de mannitol, d'acide acétique et d'acide lactique par chromatographie en utilisant une résine échangeuse de cations hautement acide;
   l'obtention d'une fraction mixte d'acide lactique et de mannitol par élution de l'acide lactique et du mannitol;
   l'obtention de mannitol sous forme de cristaux par condensation de la fraction mixte d'acide lactique et de mannitol en vue de cristalliser le mannitol; et
   l'obtention d'acide lactique sous forme de lactate de calcium par adjonction d'hydroxyde de calcium à la solution résiduelle dont le mannitol avait été éliminé.

2. Procédé de séparation un par un d'acide acétique, d'acide lactique et de mannitol comprenant :

   la condensation d'un mélange de mannitol, d'acide acétique et d'acide lactique afin de cristalliser une partie du mannitol obtenant une solution concentrée;
   la soumission de la solution condensée à une filtration afin d'éliminer en partie l'ingrédient mannitol sous forme de cristaux de mannitol et d'obtenir un filtrat;
   l'obtention dans un premier temps d'une fraction d'acide acétique par élution de l'acide acétique du filtrat obtenu contenant du mannitol, de l'acide acétique et de l'acide lactique par chromatographie en utilisant une résine échangeuse de cations hautement acide;
   l'obtention d'une fraction mixte d'acide lactique et de mannitol par élution de l'acide lactique et du mannitol;
   l'obtention de mannitol sous forme de cristaux par condensation de la fraction mixte d'acide lactique et de mannitol en vue de cristalliser le mannitol; et
   l'obtention d'acide lactique sous forme de lactate de calcium par adjonction d'hydroxyde de calcium à la solution résiduelle dont le mannitol avait été éliminé.

3. Procédé de séparation un par un d'acide acétique, d'acide lactique et de mannitol selon la revendication 1 ou 2, dans lequel la résine échangeuse de cations hautement acide est de type sodium.

4. Procédé de séparation un par un d'acide acétique, d'acide lactique et de mannitol selon la revendication 1 ou 2, dans lequel la résine échangeuse de cations est de type calcium.